# EUROPEAN PATENT APPLICATION

(11) **EP 4 644 082 A1**
(43) Date of publication of application: **05.11.2025**
(21) Application number: 24172917.7
(22) Date of filing: 29.04.2024
(51) Int. Cl.: B29C 45/00, B29C 45/14, A61N 1/375, B29K 75/00, B29K 705/00, B29L 31/00

(54) **INJECTION MOLDING OF A THERMOPLASTIC POLYURETHANE HEADER ONTO A HOUSING OF AN IMPLANTABLE MEDICAL DEVICE WITH THE AID OF A THERMALLY ACTIVATABLE POLYURETHANE FILM**

(71) Applicant: BIOTRONIK SE & Co. KG, 12359 Berlin (DE)
(72) Inventor: Egart, Boris, 13353 Berlin (DE)
(74) Representative: Biotronik Corporate Services SE

(57) **Abstract**

The present invention relates to a method for forming a header (1) on a housing (2) of an implantable medical device (100), the method comprising: providing a housing (2) having a surface (2a) to which the header (2) is to be connected, wherein a thermally activatable connecting film (3) is arranged on said surface (2a), arranging the housing (2) with respect to a mold (6) for injection molding such that said surface (2a) with the connecting film (3) thereon delimits a cavity (60) of the mold (6), and injection molding of the header (2) by filling the cavity (60) with a thermoplastic material, such that the thermally activatable connecting film (3) bonds to said surface (2a) and to the thermoplastic material.

## Description

The present invention relates to a method for forming a header of an implantable medical device and to an implantable medical device, particularly an implantable medical device having a header that has been manufactured using the method according to the present invention.

Particularly, casting of headers of active implants with cold-curing two-component epoxy resins as well as bonding of header assemblies of thermoplastic polyurethanes to titanium housing surfaces with silicone adhesives are known in the prior art.

However, processing of liquid media (such as 2-part epoxy resins or 2-part silicone adhesives) is often difficult since the use of such media cannot be easily automated. Furthermore, higher costs are often involved due to necessary mechanical rework after differential pressure casting. Further, the duration for the curing reaction is in the range of hours. Furthermore, inserts with plasma polymer layers must be heated in the injection mold to the melting temperature of the thermoplastic, resulting in a complex solution for temperature management (several heating and cooling circuits) as well as long cycle times.

Based on the above, the problem to be solved by the present invention is to provide a preferably media-tight, i.e., fluid-tight, connection between a header and a housing of an implantable medical device, particularly of components made of titanium or a titanium alloy and polyurethane in the area of the header (e.g. interface between header and housing) of an implantable medical device, particularly of an active implant.

This problem is solved by a method having the features of claim 1. Further aspects disclosed herein relate to implantable medical devices. Preferred embodiments of the method according to the present invention are stated in the dependent claims and are described below.

According to claim 1, a method for forming a header on a housing of an implantable medical device is disclosed, the method comprising:
- providing a housing of the implantable medical device, the housing having a surface to which the header is to be connected, wherein a thermally activatable connecting film is arranged on said surface,
- arranging the housing with respect to a mold for injection molding such that said surface with the connecting film thereon delimits a cavity of the mold, and
- injection molding of the header by filling the cavity with a thermoplastic material, such that the connecting film bonds to said surface and to the thermoplastic material.

The term "thermally activatable film" or "thermally activatable connecting film" is used within its meaning known to the skilled person; it particularly refers to an adhesive film that is little tacky or substantially tack-free until activated by heat. Furthermore, such thermally activatable films are also known as latent-reactive films, as for example disclosed in DE 10 2006 058527 A1. Non-limiting examples include thermally activatable adhesive polyurethane films,

This pre-application of a thermally activatable connecting film such as a thermally activatable polyurethane film to the (e.g., titanium or titanium alloy) housing surface enables the creation of the header based on injection molding with concerted formation of a sealed material composite without an additional joining step. Thus, the more complex differential pressure molding (epoxy) for forming the header may be avoided. Particularly, an adhesion mediation (sealing) between a thermoplastic material such as thermoplastic polyurethane and a titanium surface during processing of the thermoplastic material by injection molding (impact of the liquid, hot melt on the titanium surface) may be provided by the present invention in an advantageous fashion. Particularly, the thermally activatable connecting film forms a duromer (e.g., in the seconds range) under low heat and exhibits good adhesion to both partners, i.e., header and said surface of the housing.

According to an embodiment of the method, the thermally activatable connecting film is a thermally activatable polyurethane film. Particularly, in an embodiment of the method according to the present invention, the respective thermally activatable polyurethane film can be one of: DuploTEC^{®} 12233 SBF LTC or DuploTEC^{®} 12235 SBF LTC.

Furthermore, according to an embodiment, the thermoplastic material is a thermoplastic polyurethane. Particularly, said thermoplastic polyurethane is one of: Pellethane^{®} 2363-75D TPU, or Tecothane^{®} TT-1075D-M.

According to yet another embodiment of the present invention, the thermally connecting film is pressed on the surface of the housing and pre-heated to provide an initial adhesion of the film to the surface of the housing. In an embodiment, the thermally connecting film is pressed on the surface of the housing with a pressure in range of 15 N*cm⁻² to 75 N*cm⁻². Furthermore, in an embodiment, the thermally activatable connecting film is pre-heated to a temperature in the range from 45° to 65°C, preferably to a temperature of 50°C. According to yet another embodiment of the method, said pre-heating is conducted within a time period of 1s to 15s, preferably within a time period of 10s.

Furthermore, according to an embodiment of the method, the surface and/or said housing is formed out of titanium or a titanium alloy. Particularly, in an embodiment, the titanium may be a substantially pure titanium according to the standard ASTM B 265-00, i.e., the alloy comprises besides Ti, less than or equal to 0.20% Fe, less than or equal to 0.18% O, less than or equal to 0.08% C, less than or equal to 0.03% N, less than or equal to 0.015% H, wherein the rest is Ti. Particularly, the entire housing is formed out of the titanium, particularly according to said standard ASTM B 265-00.

According to a further embodiment, prior to applying the thermally activatable connecting film to the surface, the surface is cleaned. In a further embodiment, the surface is pickled. In yet another embodiment, the surface is sandblasted. In a further embodiment, the surface is wet-chemically oxidized. In yet another embodiment, the surface is laser-structured. Particularly, these processes can be combined in a desired fashion.

Furthermore, in another embodiment, prior to applying the thermally activatable connecting film and particularly prior to said pre-processing (where the surface is cleaned, pickled, sandblasted, wet-chemically oxidized, and/or laser-structured etc.), the surface is pre-treated with an adhesion promoter. In a further embodiment, the adhesion promoter is selected from the group comprised of: an alkoxysilane, a 3-aminopropyltrimethoxysilane.

Furthermore, according to yet a further embodiment of the method, the surface of the housing is adjacent an electrical feedthrough of the housing. Particularly, in an embodiment, prior to said injection molding of the header, an insert or mandrel for providing an electrical connection between a receptacle for receiving a plug (e.g., of an electrode lead) and said electrical feedthrough is connected to the feedthrough (e.g. to its pins) in an electrically conducting fashion. Particularly, the insert or mandrel may be arranged in the cavity of the mold together with the housing's surface that delimits the cavity for molding the header on the surface. Furthermore, the insert or mandrel may also be pre-fixed to the housing. However, it is also conceivable to position the insert in the mold and connect it to the feedthrough afterwards.

Particularly, according to a further embodiment of the method, said injection molding of the header further comprises embedding the insert or mandrel in the thermoplastic material to enclose the insert in the header.

Furthermore, in an embodiment of the method, the implantable medical device is one of: an implantable pacemaker, an implantable cardioverter-defibrillator, an implantable neurostimulator.

According to a further aspect of the present invention, an implantable medical device is disclosed, wherein the implantable medical device comprises a housing and a header connected a surface of the housing, wherein the header is formed on the surface of the housing using the method according to the present invention.

According to yet another aspect of the present invention, an implantable medical device is disclosed, the implantable medical device comprising a housing having a surface, the surface being formed out of titanium or a titanium alloy in particular (e.g. as described herein), wherein the implantable medical device further comprises a header, the header being injection molded from a thermoplastic material and connected to the surface via an intermediate thermally activatable connecting film, wherein particularly the thermally activatable connecting film is a polyurethane film, particularly a thermally activatable polyurethane film, and wherein in particular the thermoplastic material is a thermoplastic polyurethane.

In the following, embodiments as well as further features and advantages of the present invention shall be described with reference to the Figures, wherein
- Fig. 1: shows a connecting film being placed on a surface of a housing of an implantable medical device for generating a header according to an embodiment of the method according to the present invention;
- Fig. 2: shows a mold for injection molding arranged on the housing such that said surface of the housing with the film thereon delimits a cavity of the mold; and
- Fig. 3: shows the header connected to the housing after injection molding.

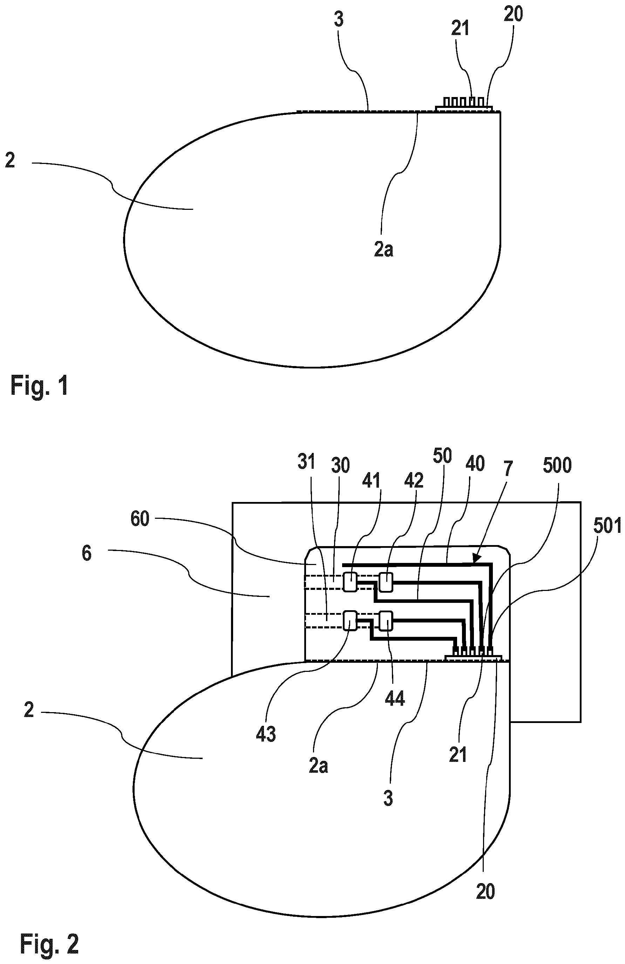

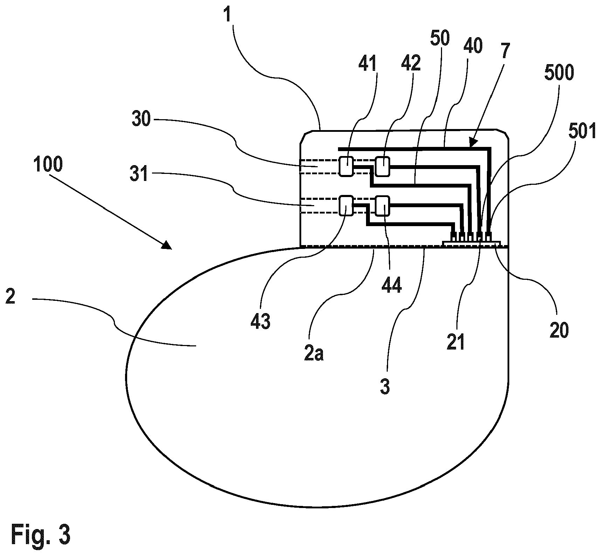

Fig. 1 shows in conjunction with Figs. 2 and 3 an embodiment of a method according to the present invention. The method allows to injection mold a header 1 of an implantable medical device 100 (cf. Fig. 3) to a housing 2 of the device 100, the housing 2 being preferably made of titanium or a titanium alloy or comprising at least a surface 2a formed out of titanium or a titanium alloy as described herein. The injection molding utilizes a thermally activatable connecting film 3, preferably a thermally activatable polyurethane film 3 that is pre-fixed to said surface 2a.

Particularly, said surface 2a and film 3 thereon on which the header 1 is later to be located delimits a cavity 60 of a mold 6 (cf. Fig. 2) used for injection molding so that the thermoplastic material that is injected into the cavity 60 of the mold 6 can make contact to the film 3 placed on said surface 2a. The sealing of the interface is achieved by the thermally activatable connecting film 3 which, under the given (injection molding) parameters such as temperature and pressure, viscosity of the melt, flow velocity, etc., remains in place, reacts (cures) and, after demolding and cooling, exhibits good adhesion to both bonding partners, i.e., header 1 and surface 2a. Particularly, injection molding may be performed with the following parameters: melt temperature (nozzle) 210°C to 225 °C, mold temperature 50°C to 70°C, holding pressure 800 bar to 1000 bar.

Particularly, the media-tight, i.e., fluid-tight, bond between the two joining partners may be produced in the following way. The housing surface 2a of the joining partner made preferably of a titanium alloy (e.g. Ex. Grade 1, ASTM B 265-00, Ra 6.3 [arithmetic mean roughness]) can be pre-processed, e.g., cleaned, particularly by automated cleaning with a mixture of water and a detergent, and subsequent rising with deionized water, pickled, particularly by treatment with a mixture with nitric acid and hydrofluoric acid to remove an oxide layer in the titanium substrate, sandblasted, wet-chemically oxidized, particularly oxidized with hydrogen peroxide and aqueous sodium hydroxide and/or, in particular, laser-structured. Laser structuring may particularly performed with the following tools and parameters: Nb:YVO₄-laser or Nb:YAG laser with a wavelength of 1064 nm (e.g. Trumpf TM6130); Q-switch 20 kHz to 100 kHz; pulse duration with 60 kHz = approx. 20 ms; P_max = 20 W (direct beam, no power losses in beam splitter, camera flange, beam scanner; spot diameter at P = 100 % and 60 kHz = approx. 70µm. In addition, the surface 2a may be pretreated with adhesion promoters from the alkoxysilane class (e.g. 3-aminopropyltrimethoxysilane, CAS No.: 13822-56-5, deposited from acetic acid ethanolic solution by dipping).

Before the joining process, the thermally activatable connecting film 3, preferably a thermally activatable polyurethane film 3 (e.g. DuploTEC^{®} 12233 SBF LTC or DuploTEC^{®} 12235 SBF LTC) is applied to the surface 2a. This may be done under brief heating (e.g., to 55 °C within about 10s) and leads to initial adhesion of the film 3 to the surface 2a so that there is no slippage during the injection molding process.

The outer geometry of the header 1 is generated by injection molding with a thermoplastic material, preferably a thermoplastic polyurethane (e.g. with Pellethane^{®} 2363-75D TPU or Tecothane^{®} TT-1075D-M).

Prior to injecting the thermoplastic material into the cavity 60 of the mold 6, and particularly prior to arranging the housing 2 with respect to the mold, an insert or header assembly 7 may be connected to an electrical feedthrough 20 of the housing 2, which feedthrough 20 is arranged adjacent said surface 2a. The insert or header assembly 7 may comprise a first and a second receptacle 30, 31 for receiving a plug (e.g., of an electrode lead), respectively. The insert or header assembly 7 may further comprise electrical contacts 41, 42, 43, 44 for contacting plugs received in the respective receptacle 30, 31. The first receptacle 30 and the second receptacle 31 may by formed by means of mandrels during injection molding, wherein the mandrels may be combined with other inlay parts, e.g., the electrical contacts 41, 42, 43, 44. Particularly, the electrical contacts 41, 42, 43, 44 may be at least partly overmolded prior to injection molding. The insert or header assembly 7 may further comprise metallic wire bands 50 that connect the contacts 41, 42, 43, 44 to feedthrough-pins 21 of the feedthrough 20 via contacts 500 of the wire bands 50. Thus, the header contacts 41, 42, 43, 44 may be connected to an electronic circuit accommodated in the housing 2 via the feedthrough 20. Furthermore, the insert or header assembly 7 may comprise an antenna 40, wherein the antenna 40 may be connected to a feedthrough-pin 21 as well via a contact 501. Particularly, the components of the insert or header assembly 7 may be separate parts but may also be connected to one another or pre-assembled in a suitable manner so that the insert or header assembly 7 as a whole may be positioned relative to the housing 2 and particularly connected thereto at once. It is conceivable to position the housing 2 together with the insert or header assembly 7 connected thereto with respect to the mold 6. However, it is also possible to position the housing 2 with respect to the mold 6 and then place the insert or header assembly 7 into the mold and connect it to the feedthrough 20.

When the thermoplastic material, e.g., thermoplastic polyurethane, is injected into the cavity 60 of the mold 6, the thermally activatable connecting film 3 (e.g. thermally activatable polyurethane film) is wetted directly with the hot melt of the thermoplastic material. Particularly, this initiates the crosslinking reaction and bonds the connecting film 3 to the surface 2a as well as to the molded thermoplastic material (e.g. thermoplastic polyurethane) of the header 1.

The present invention offers the advantage that the processing of 2-part epoxy resins that often requires manual mechanical reworking and assembly steps may be avoided. Furthermore, an automated assembly of the header is possible (including external wiring, etc.) through "design to automation". Particularly, the design of the implantable medical device enables all process steps for its assembly to be carried out in an automated ("hands-off") fashion. By reducing manual steps, the outcome of a process is more independent from an operator. Furthermore, no additional bonding steps are necessary, which allows a reduction of the total number of process steps.

## Claims

1. A method for forming a header (1) on a housing (2) of an implantable medical device (100), the method comprising:
- providing a housing (2) having a surface (2a) to which the header (2) is to be connected, wherein a thermally activatable connecting film (3) is arranged on said surface (2a),
- arranging the housing (2) with respect to a mold (6) for injection molding such that said surface (2a) with the connecting film (3) thereon delimits a cavity (60) of the mold (6), and
- injection molding of the header (2) by filling the cavity (60) with a thermoplastic material, such that the thermally activatable connecting film (3) bonds to said surface (2a) and to the thermoplastic material.

2. The method according to claim 1, wherein the thermally activatable connecting film (3) is a thermally activatable polyurethane film.

3. The method according to claim 1 or 2, wherein the thermoplastic material is thermoplastic polyurethane.

4. The method according to one of the preceding claims, wherein the thermally activatable connecting film (3) is pressed on said surface (a2 of said housing (2) and pre-heated to provide an initial adhesion of the thermally activatable connecting film (3) to the surface (2a).

5. The method according to claim 4, wherein the thermally activatable connecting film (3) is pre-heated to a temperature in the range from 45° to 65°C, preferably to a temperature of 50°C.

6. The method according to claim 4 or 5, wherein said pre-heating is conducted within a time period of 1s to 15s, preferably within a time period of 10s.

7. The method according to one of the preceding claims, wherein the surface (2a) and/or said housing (2) is formed out of titanium or a titanium alloy.

8. The method according to one of the preceding claims, wherein prior to applying the connecting film (3) to the surface (2a), the surface (2a) is one of: cleaned, pickled, sandblasted, wet-chemically oxidized, laser-structured.

9. The method according to one of the preceding claims, wherein prior to applying the thermally activatable connecting film, the surface (2a) is pre-treated with an adhesion promoter.

10. The method according to claim 9, wherein the adhesion promoter is selected from the group comprised of: an alkoxysilane, a 3-aminopropyltrimethoxysilane.

11. The method according to one of the preceding claims, wherein the surface (2a) of the housing (2) is adjacent an electrical feedthrough (20) of the housing (2).

12. The method according to claims 11, wherein prior to said injection molding of the header (2), a header assembly (7) for providing an electrical connection between a receptacle (30, 31) for receiving a plug and said electrical feedthrough (20) is connected to the electrical feedthrough (20).

13. The method according to claim 12, wherein said injection molding of the header (2) further comprises embedding the header assembly (7) in the thermoplastic material.

14. The method according to one of the preceding claims, wherein the implantable medical device (100) is one of: an implantable pacemaker, an implantable cardioverter-defibrillator, an implantable neurostimulator.

15. An implantable medical device, comprising a housing (2) and a header (1) connected to a surface (2a) of the housing (2), wherein the header (1) is formed on the surface (2a) of the housing (2) using the method according to one of the preceding claims.
